# EUROPEAN PATENT SPECIFICATION

(11) **EP 2 904 990 B1**
(45) Date of publication and mention of the grant of the patent: **08.11.2017**
(21) Application number: 15154388.1
(22) Date of filing: 09.02.2015
(51) Int. Cl.: A61F 2/30, A61F 2/32, A61F 2/34, A61F 2/36, A61F 2/38, A61F 2/40, A61F 2/42

(54) **Implant**
Implantat
Implant

(30) Priority: 07.02.2014 GB 201402164; 12.02.2014 GB 201402473
(43) Date of publication of application: 12.08.2015
(73) Proprietor: Aurora Medical Limited, Southampton, Hampshire SO16 7NS (GB)
(72) Inventor: Taylor, Andrew, Clive, Chichester, West Sussex PO18 0AW (GB)
(74) Representative: Smaggasgale, Gillian Helen

(56) References cited:
- EP-A1- 0 507 645
- DE-A1- 2 501 080
- FR-A1- 2 803 190
- GB-A- 2 476 124
- US-A1- 2004 267 375

## Description

The present invention relates to an implant. More particularly, it relates to an implant for use in an articulating joint, particularly a ball and socket joint such as a hip joint.

Joints in the body are made from two or more bones which move relative to one another and in many cases have one component which articulates against another. This is particularly the case in the ball and socket joints of the shoulder and hip. Ball and socket configurations can also be found in other joints including the knee joint and in small joints in the hands and feet.

The efficient functioning of the joints in the human body is extremely important to well-being and mobility. This is particularly the case for the hip joint, where non-functioning can severely curb the quality of life of the patient. Each hip joint comprises the upper portion of the femur which terminates in an offset bony neck surmounted by a ball-headed portion, which rotates within the acetabulum in the pelvis. Diseases such as rheumatoid- and osteoarthritis can cause erosion of the cartilage lining of the acetabulum so that the ball of the femur and the pelvis rub together, causing pain and further erosion. Bone erosion may cause the bones themselves to attempt to compensate for the erosion, which may result in the bone being reshaped. This misshapen joint may cause pain and may eventually cease to function altogether.

Operations to replace the hip joint with an artificial implant are well-known and widely practiced. Generally, the hip prosthesis will be formed of two components, namely: an acetabular, or socket, component which lines the acetabulum; and a femoral, or stem, component which replaces the femoral head. During the surgical procedure for implanting the hip prosthesis, the cartilage is removed from the acetabulum using a reamer such that it will fit the outer surface of the acetabular component of the hip prosthesis. The acetabular component can then be inserted into place. In some arrangements, the acetabular component may simply be held in place by a tight fit with the bone. However, in other arrangements, additional fixing means such as screws or bone cement may be used. The use of additional fixing means helps to provide stability in the early stages after the prosthesis has been inserted. In some modern prostheses, the acetabular component may be coated on its external surface with a bone growth promoting substance, which will assist the bone to grow and thereby assist the holding of the acetabular component in place. The bone femoral head will be removed and the femur hollowed using reamers and rasps to accept the prosthesis. The stem portion will then be inserted into the femur.

More recently, in order to reduce the amount of bone which has to be removed, the technique of femoral head resurfacing has become more widely used and, where appropriate, is replacing procedures in which stem protheses are used. In femoral head resurfacing, the ball head of the femur is machined so that a replacement surface can be provided on the machined head. In some arrangements a metal head may be used but there have also been suggestions that a ceramic material may be used.

In a conventional resurfacing procedure, the dome of the femoral head is initially removed, usually with a saw. A well is drilled into the head of the femur either before or after the dome is removed and cylinder cutters are then used to shape the femoral head until it has a cylindrical profile. At least the lower edges of the cylinder may then be chamfered.

Hip resurfacing has become an accepted method of hip replacement surgery and excellent results have been obtained, particularly for younger or more active patients where return to normal activity levels and range of motion can be expected.

However, whether a full hip replacement or resurfacing is carried out, the placement of the prosthesis is critical to reduce and preferably avoid the retained natural bony femoral neck interacting with the edge of the cup or the natural acetabulum rim, which can damage the bone and cause discomfort and even pain to the patient.

It is also essential that the prosthetic component(s) are correctly located within the body and where there are two components that they are positioned correctly relative to one another. Failure to achieve this accurate alignment may result in the patient experiencing discomfort which may be due to inappropriate frictional forces and wear. Such forces can damage the components, reduce mobility and even make a noise when the components move relative to one another. Further, it has been suggested that the friction forces may cause small particles to be released from the prosthetic, into the bloodstream. This is particularly disadvantageous if the prosthesis is formed from metal, as there have been some suggestions that these metal ions in the blood stream may have potential implications for the future well-being of the patient.

In both hip resurfacing and full hip replacement, the femoral head must be dislocated from the acetabulum and large amounts of bone and/or cartilage are removed. Not only does this subject the patient to trauma during the operation itself, the shaping of the bone and/or cartilage may increase the risk of infection and reduce the chance that the damaged bone and/or cartilage will heal. As the operation is so invasive, the operation is not generally carried out when pain is first experienced and therefore the patient may experience discomfort for many years before the operation is carried out.

Although conventional prostheses will function for many years, they generally do not have a finite life and therefore may require replacement particularly when they have been implanted in young active patients. It may therefore be necessary to carry out revision operations to replace the prosthesis. Since prostheses are firmly implanted into the bone and may be cemented into position, their removal in the revision operation can be traumatic and may result in damage to the remaining bone.

As an alternative to implanting prosthesis to replace a portion of the bone, it has been suggested that an implant can be located between the articulating bones. Examples of implants of this type are described in US2013211529 and US20090048679. These implants are configured to be inserted between the femoral head and the acetabulum and are said to improve anatomical movement.

US2009048679 describes an implant which is formed from a flexible material and has a body section forming a shell or cap with an opening and a boundary section defining the opening. The implant sits between the ball and socket of a hip joint. It is suggested that this arrangement reduces compressive strain and/or shear strain on regions of the joint surfaces affected by arthritis, as well as providing a surface with a low coefficient of friction against which the femoral head can move.

A further cap-type arrangement is described in US2013211529. Here the material from which the cap, which is located between the acetabulum and the femoral head, is manufactured swells on absorption of synovial fluid. The cap is said to create a low coefficient of friction which resists wear when it is articulated against the cartilage and/or bone of the acetabulum.

An alternative arrangement is described in WO2011/091004. Here a membrane similar in shape to a balloon is placed over the femoral head. Once it is in position, it is inflated with an inflation medium such that it provides a cushion between the acetabulum and the femoral head. The features of the preamble of claim 1 are known from FR 2803190 A1.

Whilst these arrangements offer various advantages over conventional implants and approaches for dealing with damage to joints, they still suffer from various disadvantages and drawbacks. In particular, they can still be difficult to implant. More seriously, they do not optimise the articulation of the components of the joint such that discomfort and/or further wear may still occur.

There is therefore still a need for an implant that can be readily inserted, preferably without requiring shaping of the bone and/or cartilage, and/or will provide a wide range of motion with minimal resistance.

According to the invention, there is provided an implant for an articulating two-component joint, said implant comprising a sac and a filler material located within the sac, said sac having an external surface which in use contacts bone or cartilage of the joint and which has a coefficient of friction such that it does not move relative to the bone or cartilage with which it is in contact during articulation and said filler material being a gel having a coefficient of friction that allows opposite sides of the sac to move relative to one another when a force is applied, wherein the amount of filler material is just sufficient to allow the relative movement within the sac. The configuration of the implant of the present invention means that in use there is substantially no movement between the bone or cartilage and the implant during articulation. In the implant of the present invention, all movement occurs within the filler material that is located within the sac. This enables the portion of the sac in cooperation with a first bone or cartilage component of a joint to move relative to the surface of the sac in cooperation with a second bone or cartilage component of the joint whilst not moving against the respective bone or cartilage. In particular, the implant of the present invention prevents sliding movement at the joint surfaces, which reduces friction and pain and therefore works to prevent further damage of the bone and/or cartilage. Generally the first bone or cartilage and the second bone or cartilage will be located opposed to each other in the joint.

The implant of the present invention is particularly useful in hip joints, where a wide range of motion with little resistance is desired and where dislocation of the femoral head from the acetabulum is required in known surgical methods. Thus the implant will be implanted such that one surface thereof is in contact with the acetabulum and the opposing surface is in contact with the femoral head. The implant may also be implanted in other joints, particularly those having a ball and socket configuration including the knee joint and in small joints in the hands and feet.

As the prosthesis does not move substantially against the bone and/or cartilage, further damage to the bone and/or cartilage is avoided since the implant of the present invention protects the surface of the bone and/or cartilage during subsequent articulation. In addition, the structure of the implant of the present invention may provide some cushioning between the bones which make up the joint.

A further advantage of the implant of the present invention is that it may be readily introduced without the need for major invasive surgery. In particular, the surgery required to insert the implant of the present invention will generally be minimally invasive. In a particularly preferred arrangement, the implant may be configured so that it can be delivered arthroscopically. In one arrangement, such as where the implant is to be inserted into a hip joint, incisions which are only from about 1 cm to about 2cm in length may be sufficient. It will therefore be understood that arthroscopic introduction provides substantially less trauma to a patient then implantation of a conventional prosthesis or of a revision operation. In the majority of circumstances, a general anaesthetic will not be required and insertion may be carried out under local anaesthetic. Where the joint is a hip joint, an epidural anaesthetic may be required. Being able to avoid the use of a general anaesthetic is particularly desirable with frail, elderly patients who often have pre-existing medical conditions which exacerbate the risk of adverse effects of the anaesthesia and the surgical procedure.

Since the insertion offers a low trauma approach to addressing joint pain, it is not only suitable for insertion where the patient has severe joint deterioration but may also offer advantages when inserted at an early stage. For example, where tests such as scans or x-rays indicate that deterioration of the bone or cartilage has started, the implant of the present invention may be utilised such that further damage is reduced and preferably prevented such that implantation of a full prosthesis is deferred or even avoided. Similarly the implant of the present invention may be implanted at a very early stage such as when a blood test or other assay shows markers for arthritis.

It will be understood that the implant of the present invention is suitable for implantation in any patient. For young active patients including sportsmen, the minimal invasive nature of the surgery required is beneficial as the required recovery time and risk of complications is significantly reduced when compared with conventional prosthesis implantation. The cushioning effect of the implant of the present invention may prevent further deterioration of the bones or cartilage and may even allow them to repair as they are not subjected to further wear.

The implant of the present invention offers advantages in applications where the joint has been subjected to trauma, for example in an accident, and needs time to recover and heal. The implant will allow this to occur while still allowing full patient mobility.

Some patients, particularly younger ones, may require no further intervention, other than, in certain circumstances, to remove the implant once the articulating joint has recovered although this may not be necessary.

A further advantage, particularly for senior patients is that increased mobility can be achieved without the trauma associated with conventional prosthesis insertion and without the need for a general anesthetic and its attendant risks in the older patient.

The implant of the present invention may be inserted between components which make up a natural, generally damaged, joint or between the components of a prosthetic joint.

Once inserted, the implant is generally held in position by the shape of the implant itself. Further since the insertion operation is non-invasive ligaments that hold the components of the joint together can be retained and so they will act to hold the prosthesis in position. When used in a hip joint, the implant is preferably positioned such that the tere ligament does not have to be sacrificed. Thus the optimum stability of the joint is retained.

Whilst the implant will generally be held in place by the shape of the implant and the interaction of the retained ligaments may be sufficient to hold the implant in position. In some embodiments it may be desirable to utilise a small amount of adhesive at the time of implantation. This adhesive will generally be merely a spot of a suitable biocompatible adhesive which will generally be placed in the centre of the implant.

In the implant of the present invention, the filler material is located within the sac during manufacture and the sac is sealed. There is therefore no requirement for the implant to be filled during implantation. Thus the risk of spillage or leakage of the filler material when filling the implant is obviated. Further, the controlled filling of the implant at the time of manufacture ensures that the implant is filled with the correct amount of filler and that the sterility of the material is maintained.

A still further advantage of the present invention is that use of the implant of the present invention does not require any bone and/or cartilage shaping, thereby obviating the risk of infection. The implant can also be easily repositioned relative to the components of the articulating joint. Further, if required, the implant can be carried removed and replaced. The replacement of the implant can be carried out arthroscopically.

A further advantage if the present invention is that the presence of the implant does not perturb the anatomical loading through the joint.

Since the implant of the present invention does not involve articulating metal parts, small particles, such as metal ions, are released as a result of the movement.

The sac may be formed from any suitable material. The material will generally be biocompatible or may be coated such that it is biocompatible. Generally the material will have sufficient strength to withstand implantation and the articulation of the joint into which it is implanted, while being sufficiently flexible that the implant can mould to sit against the bone or cartilage once it is in position and not prevent the desired articulation.

In one arrangement, the sac may be formed from a polymeric material. The polymeric material may be a natural or a synthetic polymer. Examples of suitable polymers include polyhydroxyalkanoates, polyurethane, silicones, and those based on cellulose.

The material from which the sac is formed may be bioresorbable. It is likely that a resorbable material will offer more advantages for younger patients so that the implant functions during the rehabilitation period but is not permanent. In contrast a permanent implant is likely to offer more advantages for the elderly patient.

In one arrangement, the surface of the implant may be smooth such that it is the coefficient of friction of the material alone which provides the friction to prevent the implant from moving with respect to the bone and/or cartilage. In one alternative, a coating may be provided on the implant to increase the friction. Additionally or alternatively, the surface of the sac may be roughened in at least some regions to increase the friction between the implant and the bone and/or cartilage. In another arrangement, the surface of the sac may be formed from a material which wrinkles as the joint articulates and these wrinkle provide the coefficient of friction required to prevent the implant moving with respect to the bone and/or cartilage.

The configuration of the implant is of a simple construction that is relatively low-cost to manufacture.

The filler material inside the sac may be any material that has the coefficient of friction required to allow opposite sides of the sac to move relative to one another when a force is applied. Generally, biocompatible materials are used. The filler material is a gel. Examples of suitable filler materials include a phospholipid or an elastomer. In one example the filler material may be a hyaluronic acid based hydrogel, a natural polymer or an alginate-based hydrogel.

The implant of the present invention may be of any suitable size and configuration. The selection of the size and configuration of the implant will generally depend on the joint into which it is to be inserted. In one arrangement, which is particularly suitable for insertion in a hip joint, the implant will be generally oval in shape although other shapes may be used. It may be from about 2 cm to about 5 cm in length and width and from about 0.5 mm to about 7mm in thickness, with thicknesses in the region of about 5 mm offering some advantages. Thicknesses of from about 2 mm, 2.5 mm, and 3 mm may be useful. In one arrangement, the implant may have a consistent thickness throughout its size and in another arrangement, it may vary in thickness. It may be thinner in a central region although in an alternative arrangement it may be thicker in the central region. The length and width of the implant will depend on the eventual use but they will generally have a thickness of about 2mm to about 5mm in thickness.

The maximum diameter of any cross-section of the implant may be less than the diameter of the equatorial plane of the convex end of a component of the joint. This allows a wider range of movement as the joint component can move further while remaining in contact with the implant. This may also reduce manufacturing costs, as less filler material may be required. In accordance with the present invention, the amount of filler will just be sufficient to allow the relative movement within the sac. Thus the opposing sides of the sac may be touching and the filler simply provides lubrication between the sides.

In one arrangement, a patient may have a pre-operative scan and the shape and size of the joint can be measured. Based on this information, the implant can be manufactured to be the optimum configuration for the patient. Thus a patient specific implant may be produced. In one arrangement, the implant of the present invention can be of a doughnut configuration. The doughnut configuration may be annular such that there is a space in the centre. Where there is space in the centre, it will not be so large that a first component of the joint does not reach through the implant since if this occurs, it may interact with another component of the joint such that wear may occur. Alternatively, it may simply be thinner than the outer region. The centre of the first component of the articulating joint may engage with the centre of the doughnut, so as to create a closer fit with the implant. In a hip joint, the articulation of the femoral head in the acetabulum does not engage across the apex of the ball but subscribes what can be described as a horse-shoe shaped path. Thus where the implant is of the doughnut configuration, the movement of the femoral head in its horse-shoe path will remain in contact with the implant.

In another arrangement, the implant may be configured to be dome-shaped when placed between the two components of the joint as this may allow a wider range of movement. Where the implant is dome-shaped, a concave side of the implant may be arranged to fit over part of a convex end of a first component of the articulating joint and a convex side of the implant opposite the concave side can move relative to the concave side.

In one alternative arrangement, the implant may further comprise a membrane, enclosed within the sac. In this arrangement, the filler material will generally be located between the membrane and the sac such that there is an area free from filler material within the membrane.

Some, or all, of the external surface of the sac may be coated with a material to facilitate bone and/or cartilage repair. The coating may allow for slow release of the repair material. Additionally or alternatively, materials which facilitate bone and/or cartilage repair may be located in the filler material and the material from which the sac is formed may allow release, preferably slow release, of the repair material. The repair material may be, or may include, stem cells.

Additionally or alternatively, the implant may be coated with pharmaceutically active material and/or the pharmaceutically active material may be located in the filler material in which case the material from which the sac is formed will allow release, preferably slow release, of the pharmaceutical material. Suitable pharmaceutical materials include those for pain relief or which are anti-bacterial. In one arrangement, the outer surface of the sac may be coated with a pharmaceutical which provides appropriate pain relief immediately after the operation and a second pharmaceutical which provides pain relief over time may be provided in the coating or within the filler of the implant. The pain relief pharmaceutical may be chosen so that the quantity delivered over time reduces.

The implant of the present invention may remain in position indefinitely or it may be removed when no longer required such that if the damage to the bone heals. Alternatively, the implant may be formed from biodegradable or bioresorbable material such that its removal after the surfaces of the articulating joints have been repaired is unnecessary as it will break down within the body.

The implant may further comprise a spacer, which may be shaped to align the bones as required or to prevent the first component of the articulating joint from contacting the other components of the articulating joint.

In one embodiment, the spacer is able to engage a component of the articulating joint and the implant. If the implant is dome-shaped, as described above, it may be the convex side of the implant that engages the spacer. This will position the component of the articulating joint away from the other components of the articulating joint, so that they do not come into contact during movement of the joint.

The spacer may be integral to the sac. This improves ease of manufacture and insertion.

In another embodiment, the spacer may be enclosed within a membrane in the implant. Again, this spacer acts to position the component of the articulating joint engaging with the implant away from the other components of the articulating joint, so that they do not come into contact during movement of the joint.

This spacer may be integral to the membrane. This improves ease of insertion. Additionally, it means that manufacture of the implant with the spacer is facilitated.

The use of a spacer may be useful when the implant is for use in a hip joint, as it positions the femoral head sufficiently far from the acetabulum that the femur and/or the femoral head will not contact the acetabulum or the pelvis during movement, which can cause discomfort and pain to the patient. The spacer may engage the acetabulum and the implant. More specifically, the spacer may engage the sac of the implant. Alternatively, the spacer may be enclosed by the membrane. The spacer should be shaped so that it can engage the concave surface of the acetabulum and provide a sufficient distance between the acetabulum and the femoral head to prevent unwanted contact but to allow the desired movement.

The spacer may not be part of the implant. Therefore, a second aspect of the present invention provides a kit of parts, including the implant of the first aspect and a spacer able to engage a component of the articulating joint and the implant. This allows a suitable spacer for the specific operation to be selected.

Whilst the implant of the present invention will generally be used with natural bone, it may offer some advantages when used with a prosthesis. For example, the implant may be used to provide relief when a previously implanted prosthesis is exhibiting wear. Since the implant of the present invention can be readily implanted without the need for substantial surgery, the implant may be used to provide temporary relief or to defer the need for revision surgery.

There may also be some advantages in including an implant of the present invention between a newly implanted prosthetic component and a natural bone component of a joint or between two prosthetic components.

Although the implant of the present invention may be separate from the bones and/or cartilage, in one arrangement, one or more skirts may be attached to the implant and which, in use, sit in contact with the respective bone and/or cartilage. Where present the, or each skirt, will generally be formed from the same material as used to form the sac and may be integral therewith. Where present the, or each, skirt merely acts to site the implant in position. The connection of the skirt to the implant is configured in such a manner that it does not alter the functioning of the implant. In one arrangement where the joint is a hip joint, the skirt may be configured to sit around the femoral head. The skirt may include apertures or cutaway portions to accommodate ligaments.

The present invention will now be described, by way of example, with reference to the accompanying figures in which:
- Figure 1: is a schematic representation of the implant of the present invention in cross-section, in position in a hip joint;
- Figure 2: is a perspective view from above of the implant before implantation in the body;
- Figures 3a to c: are schematic representations illustrating the movement of the implant during articulation of the hip joint;
- Figure 4: is a perspective view from above of the implant having a domed configuration and an enlarged portion thereof;
- Figure 5: illustrates the likely location of the implant in a hip joint;
- Figure 6: is an enlarged view of portion B from Figure 5;
- Figure 7: is a schematic representation of an implant in use with a spacer;
- Figure 8: is a close up of portion C from Figure 7;
- Figure 9: is a schematic representation of one alternative implant;
- Figure 10: is a close up of portion B of Figure 9;
- Figure 11: is a schematic representation of a second alternative implant;
- Figure 12: is a schematic representation of a third alternative implant;
- Figure 13: is a schematic representation of a fourth alternative implant; and

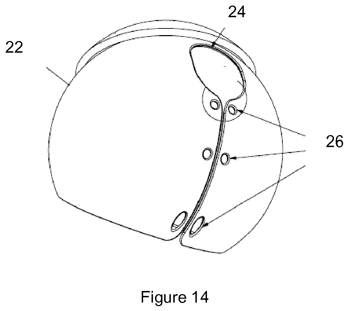

- Figure 14: is a schematic representation of a fifth alternative implant.

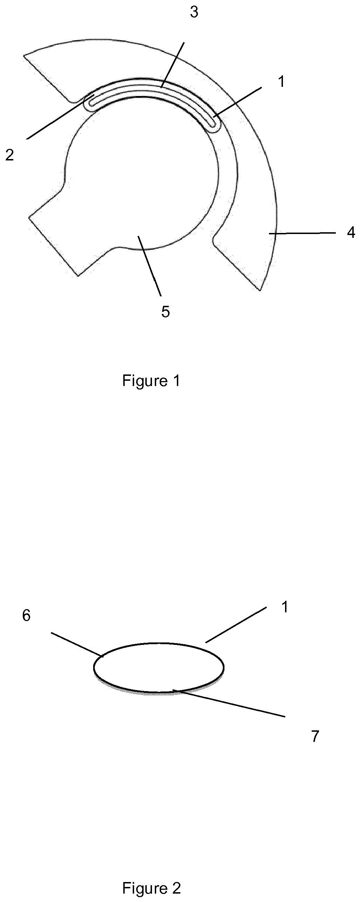

As illustrated in Figure 1, the implant 1 of the present invention comprises a sac 2 containing a filler material 3. It will be understood that the implant is illustrated in cross-section and that the sac 2 completely encloses the filler material. It will also be understood that the figure is not to scale and the thickness of the filler may just be sufficient to enable the opposing surfaces of the sac to move relative to one another. The implant 1 is illustrated located between the femoral head 5 and the acetabulum 4 of the hip joint. Again it will be understood that these are not drawn to scale. Although the implant has been illustrated in a hip joint this is for ease of illustration. The implant may be used in other ball and socket joints.

A perspective view of the implant 1 is illustrated in Figure 2. The implant 1 comprises a rim 6 and central region 7. The implant may be of consistent thickness throughout the implant or may be thicker or thinner in the central region 7 than at the rim 6.

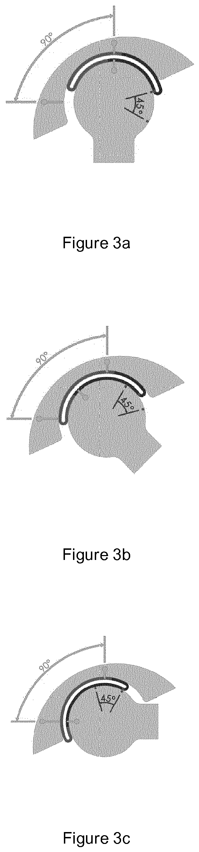

Figures 3a, 3b and 3c provide a schematic representation showing how a particular point on the surface of the implant does not move against the portion of the bone with which it is in contact during articulation of the bone. Instead opposing surfaces of the implant move relative to one another as facilitated by the low-coefficient of friction of the filler material. In this illustration, the movement during articulation has been exaggerated as has the size of the implant in order to facilitate viewing of the movement. Movement has only been illustrated in two dimensions although it will be understood that the movement will occur in three dimensions.

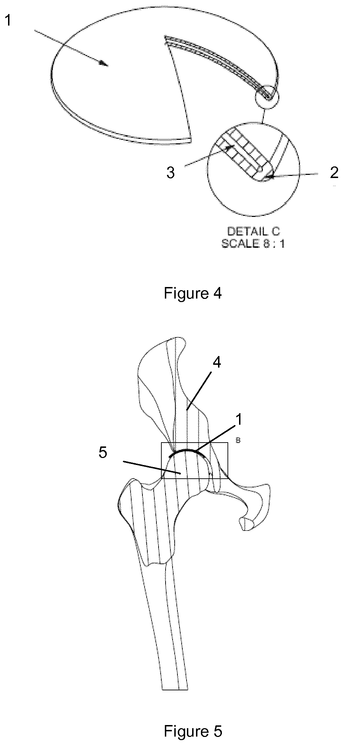

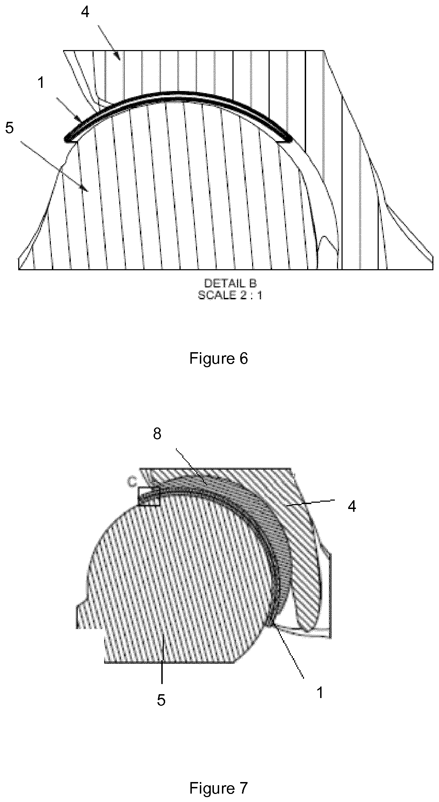

As illustrated in Figure 4, the implant 1 may have a dome shape. The final shape of the implant will depend on the joint into which it is to be implanted. Where the implant 1 is located in a hip joint, it may be positioned as illustrated in Figures 5 and 6.

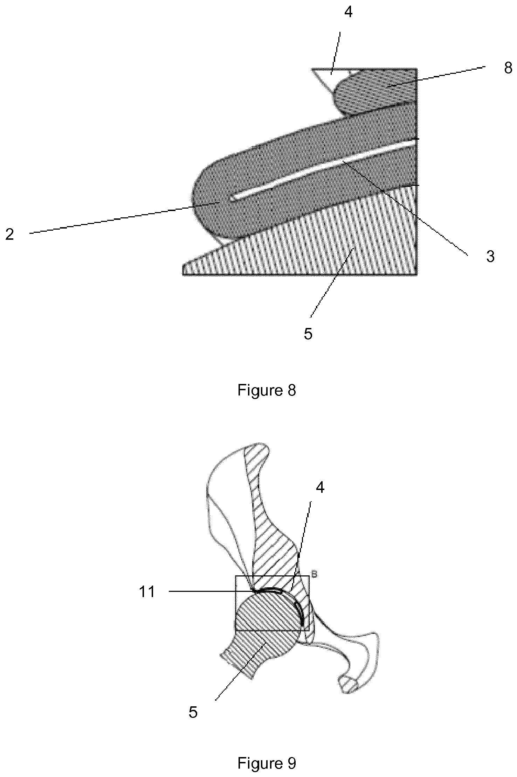

As illustrated in Figure 7 the implant of the present invention may be used in combination with a spacer 8. The spacer may be a connected to the implant or may be separate therefrom. The region C is illustrated in an enlarged section in Figure 8.

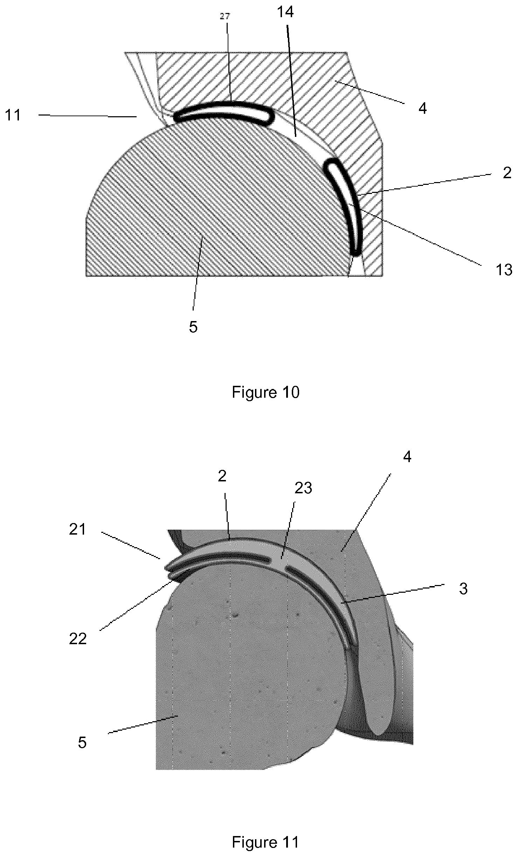

An alternative embodiment of the implant is illustrated in Figures 9 and 10. In this arrangement, the implant 11 is of an annular or donut configuration. In the illustrated arrangement, the implant has an aperture 14 in the centre. The implant 11 comprises a sac 12 and a filler 13.

An alternative configuration of the implant of the present invention is illustrated in Figure 11. In this arrangement, a skirt 22 is extends from the implant 21 and is connected thereto by a lug 23. The skirt 22 in use sits on the bone. In the illustrated arrangement, it sits on the femoral head. The skirt 22 and the lug 23 are generally formed from the same material as the sac 2 and may be moulded therewith. The lug is small in size and does not affect the operation of the implant.

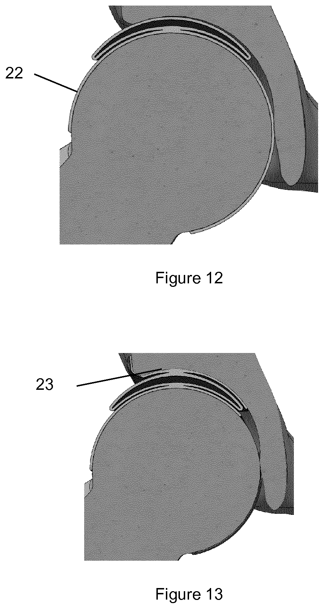

The skirt illustrated in Figure 11 extends on the femoral head for a distance which is of a similar size to the size of the sac. In an alternative arrangement the skirt may be larger and may, in one arrangement, extend around the head of the femur as illustrated in Figure 13.

In a still further alternative configuration, a second skirt 23 extends from the side of the implant opposite to the first skirt 22. In use this skirt 23 sits against the acetabulum.

In one arrangement, the skirt 22 includes an aperture 24 to accommodate the tere ligament. The skirt may include a slit 25 which enables it to be inserted on the femoral head around the ligament. Holes 26 may be included around the slit 25. Once in position clips, not shown, may be inserted to hold the skirt in position.

## Claims

1. An implant for an articulating two-component joint, said implant (1) comprising a sac (2) and a filler material (3) located within the sac, said sac having an external surface which in use contacts bone or cartilage of the joint and which has a coefficient of friction such that it does not move relative to the bone or cartilage with which it is in contact during articulation and said filler material being a gel having a coefficient of friction that allows opposite sides of the sac (2) to move relative to one another when a force is applied, **characterised in that** the amount of filler material is just sufficient to allow the relative movement within the sac.

2. An implant according to Claim 1 configured to be delivered arthroscopically.

3. An implant according to Claim 1 or 2 wherein the sac (2) is formed from a natural or a synthetic polymer.

4. An implant according to Claim 3 wherein the polymer is polyhydroxyalkanoates, polyurethane, silicones, or those based on cellulose.

5. An implant according to any one of Claims 1 to 4 wherein the sac (2) is formed from bioresorbable polymer.

6. An implant according to any one of Claims 1 to 5 wherein a coating may be present on the surface of the implant (1) to provide friction.

7. An implant according to any one of Claims 1 to 6 wherein the filler material (3) is selected from a phospholipid, an elastomer, a hyaluronic acid based hydrogel, a natural polymer and an alginate-based hydrogel.

8. An implant according to any one of Claims 1 to 7 wherein the implant (1) is from 0.5mm to 7mm in thickness.

9. An implant according to Claim 8 wherein the implant (1) is from 5mm in thickness.

10. An implant according to any one of Claims 1 to 9 wherein the implant (1) is patient specific.

11. An implant according to any one of Claims 1 to 10 the implant (1) is of a doughnut configuration.

12. An implant according to Claim 11 wherein there is a space in the centre of the doughnut configuration.

13. An implant according to Claim 12 wherein the implant (1) is dome-shaped.

14. An implant according to any one of Claims 1 to 13 wherein the external surface of the sac (2) is coated with a material to facilitate bone and/or cartilage repair and/or the material which facilitates bone and/or cartilage repair is located in the filler material (3).

15. An implant according to Claim 14 wherein the repair material is stem cells.

16. An implant according to any one of Claims 1 to 15 wherein the implant (1) is coated with pharmaceutically active material and/or pharmaceutically active material may be located in the filler material.

17. An implant according to Claim 16 wherein the pharmaceutically active material is slow release.

18. An implant according to any one of Claims 1 to 17 additionally comprising a spacer.

19. An implant according to any one of Claims 1 to 18 additionally including one or more skirts attached to the implant and which, in use, sit in contact with the respective bone and/or cartilage.

## Patentansprüche

1. Implantat für eine Zweikomponenten-Gelenkverbindung, wobei das Implantat (1) einen Beutel (2) und ein Füllmaterial (3), das in dem Beutel vorliegt, umfasst, wobei der Beutel eine Außenoberfläche aufweist, die bei Verwendung mit dem Knochen oder Knorpel des Gelenks im Kontakt ist und die einen Reibungskoeffizienten aufweist, sodass er sich relativ zum Knochen oder Knorpel, mit dem er während der Gelenkbildung im Kontakt ist, nicht bewegt, und das Füllmaterial ein Gel ist, das einen Reibungskoeffizienten aufweist, der ermöglicht, dass gegenüberliegende Seiten des Beutels (2) sich relativ zueinander bewegen, wenn eine Kraft angewendet wird, **dadurch gekennzeichnet, dass** die Menge an Füllmaterial gerade ausreicht, um die relative Bewegung innerhalb des Beutels zu ermöglichen.

2. Implantat nach Anspruch 1, das zur arthroskopischen Abgabe konfiguriert ist.

3. Implantat nach Anspruch 1 oder 2, wobei der Beutel (2) aus einem natürlichen oder einem synthetischen Polymer gebildet ist.

4. Implantat nach Anspruch 3, wobei das Polymer Polyhydroxyalkanoate, Polyurethan, Silikone oder solche auf Cellulose basierte ist.

5. Implantat nach einem der Ansprüche 1 bis 4, wobei der Beutel (2) aus bioresorbierbarem Polymer gebildet ist.

6. Implantat nach einem der Ansprüche 1 bis 5, wobei eine Beschichtung auf der Oberfläche des Implantats (1) vorliegen kann, um Reibung bereitzustellen.

7. Implantat nach einem der Ansprüche 1 bis 6, wobei das Füllmaterial (3) aus einem Phospholipid, einem Elastomer, einem auf Hyaluronsäure basierten Hydrogel, einem natürlichen Polymer und einem Alginat-basierten Hydrogel ausgewählt ist.

8. Implantat nach einem der Ansprüche 1 bis 7, wobei das Implantat (1) eine Dicke von 0,5 mm bis 7 mm aufweist.

9. Implantat nach Anspruch 8, wobei das Implantat (1) eine Dicke von 5 mm aufweist.

10. Implantat nach einem der Ansprüche 1 bis 9, wobei das Implantat (1) patientenspezifisch ist.

11. Implantat nach einem der Ansprüche 1 bis 10, wobei das Implantat (1) eine Doughnut-Konfiguration aufweist.

12. Implantat nach Anspruch 11, wobei ein Raum in der Mitte der Doughnut-Konfiguration vorliegt.

13. Implantat nach Anspruch 12, wobei das Implantat (1) kuppelförmig ist.

14. Implantat nach einem der Ansprüche 1 bis 13, wobei die Außenoberfläche des Beutels (2) mit einem Material beschichtet ist, um Knochen- und/oder Knorpelreparatur zu unterstützen, und/oder das Material, das Knochen- und/oder Knorpelreparatur unterstützt, in dem Füllmaterial (3) vorliegt.

15. Implantat nach Anspruch 14, wobei das Reparaturmaterial Stammzellen ist.

16. Implantat nach einem der Ansprüche 1 bis 15, wobei das Implantat (1) mit pharmazeutischem Wirkmaterial beschichtet ist und/oder pharmazeutisches Wirkmaterial in dem Füllmaterial vorliegen kann.

17. Implantat nach Anspruch 16, wobei das pharmazeutische Wirkmaterial langsam abgegeben wird.

18. Implantat nach einem der Ansprüche 1 bis 17, das zusätzlich einen Abstandhalter umfasst.

19. Implantat nach einem der Ansprüche 1 bis 18, das zusätzlich eine oder mehrere Einfassungen umfasst, die an dem Implantat angebracht sind und die bei Verwendung im Kontakt mit dem jeweiligen Knochen und/oder Knorpel sitzen.

## Revendications

1. Implant pour une articulation à deux composants articulés, ledit implant (1) comprenant un sac (2) et un matériau de remplissage (3) situé au sein du sac, ledit sac ayant une surface externe qui pendant l'utilisation entre en contact avec l'os ou le cartilage de l'articulation et qui a un coefficient de frottement tel qu'elle ne se déplace pas par rapport à l'os ou au cartilage avec lequel elle est en contact pendant l'articulation et ledit matériau de remplissage étant un gel ayant un coefficient de frottement qui permet aux côtés opposés du sac (2) de se déplacer l'un par rapport à l'autre quand une force est appliquée, **caractérisé en ce que** la quantité de matériau de remplissage est juste suffisante pour permettre le mouvement relatif au sein du sac.

2. Implant selon la revendication 1 configuré pour être délivré de manière arthroscopique.

3. Implant selon la revendication 1 ou 2 dans lequel le sac (2) est formé à partir d'un polymère naturel ou synthétique.

4. Implant selon la revendication 3 dans lequel le polymère est sélectionné parmi des polyhydroxyalcanoates, du polyuréthane, des silicones, ou des polymères à base de cellulose.

5. Implant selon l'une quelconque des revendications 1 à 4 dans lequel le sac (2) est formé à partir d'un polymère biorésorbable.

6. Implant selon l'une quelconque des revendications 1 à 5 dans lequel un revêtement peut être présent sur la surface de l'implant (1) pour fournir un frottement.

7. Implant selon l'une quelconque des revendications 1 à 6 dans lequel le matériau de remplissage (3) est sélectionné parmi un phospholipide, un élastomère, un hydrogel à base d'acide hyaluronique, un polymère naturel et un hydrogel à base d'alginate.

8. Implant selon l'une quelconque des revendications 1 à 7 dans lequel l'implant (1) mesure de 0,5 mm à 7 mm d'épaisseur.

9. Implant selon la revendication 8 dans lequel l'implant (1) mesure 5 mm d'épaisseur.

10. Implant selon l'une quelconque des revendications 1 à 9 dans lequel l'implant (1) est spécifique au patient.

11. Implant selon l'une quelconque des revendications 1 à 10 dans lequel l'implant (1) a une configuration torique.

12. Implant selon la revendication 11 dans lequel il y a un espace au centre de la configuration torique.

13. Implant selon la revendication 12 dans lequel l'implant (1) est en forme de dôme.

14. Implant selon l'une quelconque des revendications 1 à 13 dans lequel la surface externe du sac (2) est revêtue d'un matériau pour faciliter la réparation de l'os et/ou du cartilage et/ou le matériau qui facilite la réparation de l'os et/ou du cartilage est situé dans le matériau de remplissage (3).

15. Implant selon la revendication 14 dans lequel le matériau de réparation est des cellules souches.

16. Implant selon l'une quelconque des revendications 1 à 15 dans lequel l'implant (1) est revêtu d'un matériau pharmaceutiquement actif et/ou un matériau pharmaceutiquement actif peut être situé dans le matériau de remplissage.

17. Implant selon la revendication 16 dans lequel le matériau pharmaceutiquement actif est à libération prolongée.

18. Implant selon l'une quelconque des revendications 1 à 17 comprenant en outre une pièce d'espacement.

19. Implant selon l'une quelconque des revendications 1 à 18 incluant en outre une ou plusieurs jupes attachées à l'implant et qui, pendant l'utilisation, sont posées en contact avec l'os et/ou le cartilage respectifs.
